# EUROPÄISCHE PATENTSCHRIFT

(11) **EP 1 721 584 B1**
(45) Veröffentlichungstag und Bekanntmachung des Hinweises auf die Patenterteilung: **20.02.2008**
(21) Anmeldenummer: 06005930.0
(22) Anmeldetag: 23.03.2006
(51) Int. Cl.: A61F 2/38

(54) **Knieendoprothese mit einem Beugescharnier**
Knee endoprosthesis with a bending hinge
Endoprothèse de genou avec une charnière à flexion

(30) Priorität: 09.05.2005 DE 102005022583
(43) Veröffentlichungstag der Anmeldung: 15.11.2006
(73) Patentinhaber: AESCULAP AG & Co. KG, 78532 Tuttlingen (DE); Reignier, Bernard, 72170 Maresché (FR)
(72) Erfinder: Roussel, Charly, 52000 Chaumont (FR); Reignier, Bernard, 72170 Maresché (FR)
(74) Vertreter: Hoeger, Stellrecht & Partner Patentanwälte

(56) Entgegenhaltungen:
- EP-A- 1 417 938
- FR-A- 2 696 926
- US-A- 5 282 867
- US-A1- 2004 186 584

## Beschreibung

Die Erfindung betrifft eine Knieendoprothese mit einem Tibiateil und einem Femurteil, die über ein Scharniergelenk miteinander schwenkbar verbunden sind, welches eine in zwei seitlichen Lageröffnungen des Femurteils gelagerte Lagerwelle und diese Lageröffnungen außenseitig verschließende Deckel umfaßt.

Bei Knieendoprothesen vom Scharniertyp werden ein Femurteil der Knieprothese und ein Tibiateil der Knieprothese über eine Lagerwelle schwenkbar miteinander verbunden, die üblicherweise in Lageröffnungen des Femurteils gelagert ist.

Es gibt dabei Konstruktionen, bei denen die Lageröffnungen zur Außenseite hin offen bleiben, die Lagerwelle verschließt also die Lageröffnungen von innen und ist von außen frei zugänglich (US 5,370,701; EP 0 400 045 B1).

Dabei ist zwar eine leichte Zugänglichkeit zur Lagerwelle gegeben, falls dies beispielsweise bei einer Reoperation gewünscht wird, andererseits besteht aber die Gefahr, daß durch die seitlich offene Lageröffnung Knochensubstanz und andere Körpersubstanzen in die Lageröffnungen eindringen und dort zu Schädigungen Anlaß geben, außerdem ist es bei diesen Konstruktionen notwendig, die Lagerwelle in den Lageröffnungen in axialer Richtung zu sichern.

Es ist auch eine Knieendoprothese des Scharniertyps bekannt, bei der die Lagerwelle einstückig mit einem Gewindestopfen verbunden ist, der in eine der beiden Lageröffnungen einschraubbar ist. In die andere Lageröffnung ist ein entsprechender Gewindestopfen einschraubbar, der eine Lagerausnehmung für die Lagerung der Lagerwelle aufweist (FR 2 696 926). Bei dieser Konstruktion bilden die Gewindestopfen die Lagerwelle und die eigentliche Lageröffnung der Lagerwelle aus, auf einer Seite ist ein Zugang zur Lagerwelle nur möglich, wenn diese zusammen mit dem Gewindestopfen aus der Lageröffnung des Femurteils herausgeschraubt wird, da sie einstückig mit diesem Gewindestopfen verbunden ist, in beiden Fällen ist das Einsetzen und Entfernen der Gewindestopfen mit einer großen Zahl von Umdrehungen verbunden und daher während einer Operation außerordentlich umständlich.

Es ist insbesondere bei Reoperationen wünschenswert, den Zugang zur Lagerwelle freizugeben, um dort gegebenenfalls notwendige Justierungen oder einen Austausch von Teilen vornehmen zu können, andererseits ist es wünschenswert, wenn die Lageröffnungen außenseitig dicht verschlossen sind, um das Eindringen unerwünschter Substanzen zu verhindern und um gegebenenfalls auch die Lagerwelle in axialer Richtung zu sichern.

Es ist Aufgabe der Erfindung, bei einer gattungsgemäßen Knieendoprothese eine Möglichkeit zu schaffen, die Lageröffnungen des Femurteils in konstruktiv einfacher Weise zu verschließen und gegebenenfalls wieder öffnen zu können.

Diese Aufgabe wird bei einer Knieendoprothese der eingangs beschriebenen Art erfindungsgemäß dadurch gelöst, daß die Deckel mittels eines Bajonettverschlusses lösbar in der jeweiligen Lageröffnung gehalten sind und zwischen einer Freigabestellung, in der sie aus der Lageröffnung in axialer Richtung herausziehbar beziehungsweise in diese einschiebbar sind, und einer Schließstellung, in der sie in der Lageröffnung in axialer Richtung festgelegt sind, verdrehbar sind.

Die Verwendung eines Deckels, der mittels eines Bajonettverschlusses in der Lageröffnung gehalten ist, führt dazu, daß mit einer Umdrehung des Deckels um deutlich weniger als einer Vollumdrehung dieser aus der Schließstellung in die Öffnungsstellung und umgekehrt gebracht werden kann, so daß es außerordentlich einfach ist, den Deckel bei Bedarf einzusetzen beziehungsweise abzunehmen. Außerdem erreicht man dadurch eine sehr geringe Bauhöhe, da eine große Anzahl von Gewindegängen wie bei vorbekannten Lösungen nicht notwendig ist, mittels eines Bajonettverschlusses kann ein Deckel auch in einer sehr dünnen Lagerwand des Femurteils ohne weiteres gehalten werden.

Gemäß einer bevorzugten Ausführungsform ist vorgesehen, daß der Bajonettverschluß mindestens zwei sich über einen Teil des Umfanges längs des Randes der Lageröffnung erstreckende, zur Innenseite der Lageröffnung hin offene Nuten umfaßt, die in axialer Richtung an einem Ende außenseitig eine Einführöffnung aufweisen, und daß der Deckel für jede Nut einen radialen Vorsprung trägt, der beim winkelrichtigen Aufsetzen des Deckels auf die Lageröffnung in dessen Freigabestellung in axialer Richtung durch die Einführöffnung einer Nut in die Ebene der Nut vorschiebbar ist und beim Verdrehen des Deckels in die Nut eintritt und in dieser verschoben wird. Durch das Vorsehen einer solchen randparallelen Nut wird eine genau definierte Führung des Deckels beim Verdrehen zwischen der Freigabestellung und der Schließstellung gewährleistet, außerdem wird der Deckel in seiner Ebene, das heißt in axialer Richtung, zuverlässig gehalten.

In der Regel ist es ausreichend, wenn zwei derartige Nuten an der Lageröffnung diametral gegenüberliegen und der Deckel zwei entsprechende radiale Vorsprünge trägt.

Vorzugsweise erstrecken sich die Nuten über einen Umfangswinkel von maximal 90°, so daß auch der Drehwinkel des Deckels beim Verdrehen zwischen der Schließstellung und der Freigabestellung nicht größer ist.

Gemäß einer bevorzugten Ausführungsform kann vorgesehen sein, daß der Deckel und die Lageröffnung in der Schließstellung durch ein elastisch in eine Rastausnehmung eintauchendes Rastglied gegen eine Verdrehung lösbar gesichert sind. Einerseits wird dadurch sichergestellt, daß der Deckel sich nicht unbeabsichtigt in die Freigabestellung verdreht, andererseits spürt der Operateur beim Verdrehen des Deckels in die Schließstellung, wann der Deckel die Schließstellung erreicht hat, dies läßt sich nämlich am elastischen Einschnappen des Rastgliedes in die Rastausnehmung feststellen.

Günstig ist es, wenn das Rastglied durch die radialen Vorsprünge und die Rastausnehmungen durch Vertiefungen im Boden der Nuten an deren den Einführöffnungen abgewandten Enden gebildet werden. Bei einer solchen Ausgestaltung übernehmen also die radialen Vorsprünge eine Doppelfunktion, einmal nämlich die Funktion der den Deckel in axialer Richtung haltenden Vorsprünge, und zum anderen die Funktion der elastisch in Rastausnehmungen einschnappbaren Rastglieder.

Es ist günstig, wenn die Rastglieder und die Rastausnehmungen einen komplementären, bogenförmigen Querschnitt aufweisen, so daß unter Überwindung der elastischen Rückhaltekraft auch ohne weiteres wieder eine Verdrehung des Deckels in die Freigabestellung möglich ist.

Bei einer bevorzugten Ausführungsform ist vorgesehen, daß die Rastglieder an elastisch verformbaren Haltearmen angeordnet und in radialer Richtung federnd nach innen eindrückbar sind. Dadurch erhält man bei kleiner Baugröße einen relativ großen Federweg.

Die Haltearme können insbesondere an einem Träger außenseitig angeformt sein, der an der Innenseite eines Deckels festgelegt ist.

Dieser Träger kann als Ring ausgebildet sein.

In diesem Falle ist es günstig, wenn der Ring drehfest auf einen Haltezapfen auf der Innenseite des Deckels aufgesteckt ist.

Gemäß einer bevorzugten Ausführungsform erstrecken sich die Haltearme im wesentlichen konzentrisch zum Rand des Deckels über einen Teil seines Umfanges, so daß eine sehr große Länge der Haltearme möglich ist und damit eine besonders gute Federwirkung.

Der Träger, die Haltearme und die Rastvorsprünge können vorzugsweise Teile eines einstückig ausgebildeten Bauteils sein, es kann sich dabei beispielsweise um ein plattenförmiges Materialstück handeln.

Der Deckel kann auf seiner Außenseite außermittige Vertiefungen zum manuellen Erfassen oder zum Ansetzen eines Drehwerkzeuges aufweisen.

Günstig ist es, wenn der Deckel mit seiner Außenseite die Lageröffnung bündig verschließt, so daß an keiner Stelle Vorsprünge oder Spalte entstehen, die umgebendes Gewebe verletzen oder das Eindringen von umgebendem Gewebe begünstigen könnten.

Die nachfolgende Beschreibung bevorzugter Ausführungsformen der Erfindung dient im Zusammenhang mit der Zeichnung der näheren Erläuterung. Es zeigen:

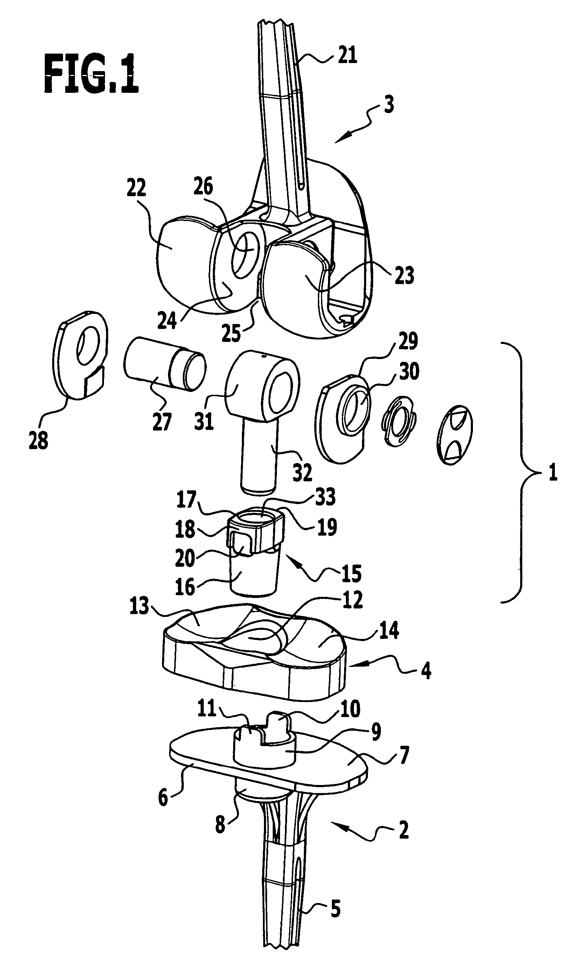

- **Figur 1:**: eine perspektivische Explosionsansicht einer gekoppelten Knie-endototalprothese;

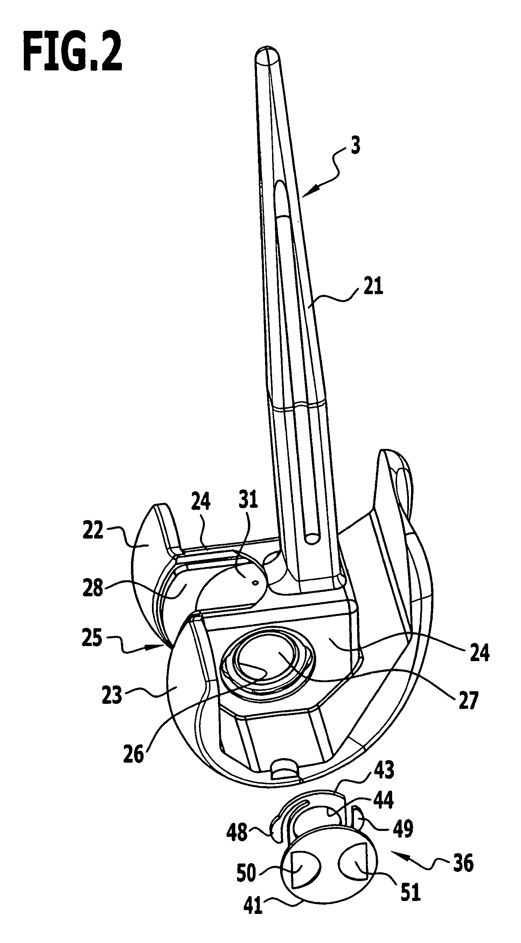

- **Figur 2:**: eine perspektivische Ansicht des Femurteils der Prothese der Figur 1 mit zerlegtem und herausgenommenem Deckel für die Lageröffnung;
- **Figur 3:**: eine vergrößerte perspektivische Ansicht der Lageröffnung der Prothese der Figuren 1 und 2 ohne eingesetzten Deckel;

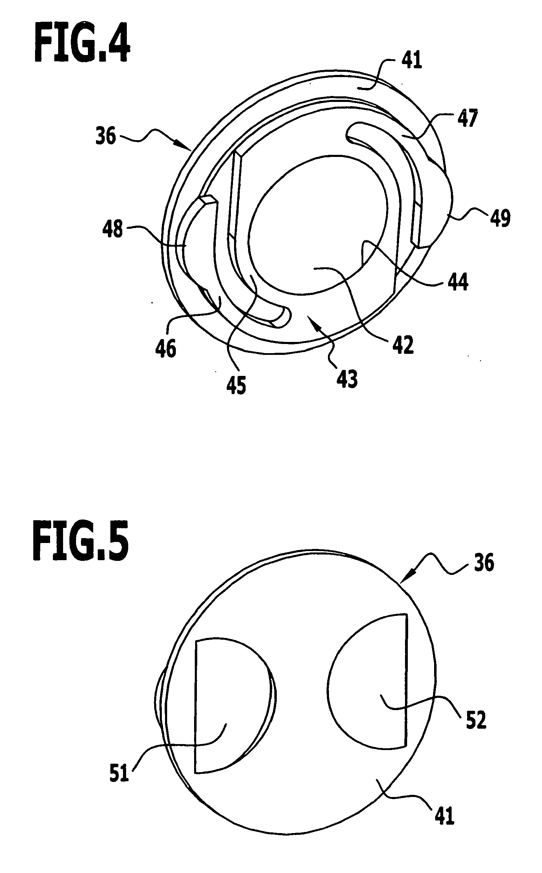

- **Figur 4:**: die Rückseite eines Deckels für die Lageröffnung und
- **Figur 5:**: die Vorderseite eines Deckels für die Lageröffnung.

Die Erfindung wird nachstehend am Beispiel einer gekoppelten Knieendototalprothese erörtert, es versteht sich aber, daß die beschriebene Konstruktion auch für Knieprothesen anderer Bauart Verwendung finden kann, sofern die Prothese als Scharnierprothese mit einer Lagerwelle ausgebildet ist, deren Enden in Lageröffnungen eines Femurteils gelagert sind.

Die in der Zeichnung dargestellte Knieendoprothese 1 umfaßt ein Tibiateil 2, ein Femurteil 3 und ein zwischen den beiden angeordnetes Meniskusteil 4.

Am Tibiateil 2 ist an der Oberseite eines Haltezapfens 5, der in den Markraum eines Tibiaknochens eingeschoben wird, eine quer zur Längsrichtung des Haltezapfens 5 verlaufende Tibiaplatte 6 gehalten, deren ebene Oberseite eine Lagerfläche 7 für das an seiner Unterseite ebene Meniskusteil 4 bildet. Die Tibiaplatte 6 wird von einer zylindrischen Aufnahmebuchse 8 durchsetzt, deren Längsachse senkrecht zur Lagerfläche 7 verläuft und die die Lagerfläche 7 in Form eines Ringflansches 9 überragt. Auf zwei gegenüberliegenden Seiten trägt dieser Ringflansch 9 zwei nach oben abstehende Vorsprünge 10, 11.

Das Meniskusteil 4, das im Gegensatz zu dem metallischen Tibiateil 2 und dem metallischen Femurteil 3 aus einem Kunststoff besteht, insbesondere aus Polyethylen, überdeckt im wesentlichen die gesamte Lagerfläche 7 und weist eine zentrale, im Querschnitt kreisförmige Durchbrechung 12 auf, die mit ihrer Innenwand an der Außenseite des Ringflansches 9 dicht anliegt, so daß der Ringflansch 9 ein Drehgelenk für das Meniskusteil 4 ausbildet, dieses ist somit auf der Lagerfläche 7 um eine Drehachse verdrehbar, die mit der Längsachse der Aufnahmebuchse 8 zusammenfällt.

Zu beiden Seiten der Durchbrechung 12 sind auf der Oberseite des Meniskusteils 4 jeweils eine konkave Lagerschale 12, 13 eingearbeitet.

In die Aufnahmebuchse 8 ist von oben her eine Lagerhülse 15 eingesteckt, die einen zylindrischen unteren Abschnitt 16 und einen annähernd quaderförmigen Kopf 17 aufweist. An zwei gegenüberliegenden Seiten 18, 19 des Kopfes sind Ausnehmungen 20 eingearbeitet, die in ihrer Form komplementär zu den Vorsprüngen 10, 11 ausgebildet und nach unten offen sind. Beim Einstecken der Lagerhülse 15 in die Aufnahmebuchse 8 treten die Vorsprünge 10, 11 in die entsprechenden Ausnehmungen 20 des Kopfes 17 ein und legen dadurch die Lagerhülse 15 drehfest in der Aufnahmebuchse 8 fest. Der Kopf 17 ragt dabei über das Meniskusteil 4 nach oben hervor.

Das Femurteil 3 weist in ähnlicher Weise wie das Tibiateil 2 einen Haltezapfen 21 auf, der in den Markraum des Femurknochens eingeschoben wird, und der an einem Ende zwei im Abstand zueinander und parallel zueinander verlaufende gebogene Gleitflächen 22, 23 trägt, die bei montierter Knieendoprothese in die Lagerschalen 13, 14 eintauchen und sich in diesen abstützen.

Zwischen den beiden Gleitflächen 22, 23 ist eine durch zwei senkrechte Seitenwände 24 begrenzte Lagerkammer 25 ausgebildet. Die beiden Seitenwände 24 weisen je eine Lageröffnung 26 auf, die der Lagerung einer die Lagerkammer 25 quer durchsetzenden Lagerwelle 27 dienen. Zur Lagerung der Lagerwelle 27 werden zwei Lagerscheiben 28, 29 auf die Lagerwelle 27 aufgesteckt, diese umgeben die Lagerwelle 27 mit einem seitlich von den Lagerscheiben 28, 29 abstehenden ringflanschförmigen Bund 30, der in die Lageröffnungen 26 hineinragt und somit die Lagerwelle definiert in den Lageröffnungen 26 hält. Diese Lagerscheiben sind vorzugsweise aus einem Kunststoff gefertigt, beispielsweise aus Polyethylen.

Zwischen den beiden Lagerscheiben 28, 29 ist auf der Lagerwelle 27 mittels eines die Lagerwelle 27 umgebenden Lagerringes 31 ein radial von diesem abstehender Lagerzapfen 32 gelagert, der in den komplementär zu ihm ausgebildeten Innenraum 33 der Lagerhülse 15 eintaucht. Auf diese Weise werden das Femurteil 3 einerseits und das Tibiateil 2 andererseits über ein Scharniergelenk miteinander schwenkbar verbunden. Dieses Scharniergelenk ist jedoch in seiner Höhe relativ zum Tibiateil 2 verschiebbar, da der Lagerzapfen 32 in der Lagerhülse 15 in axialer Richtung frei verschiebbar ist. Der Abstand von Femurteil 3 und Tibiateil 2 wird durch die Anlage der Gleitflächen 22 und 23 an den Lagerschalen 13 beziehungsweise 14 bestimmt.

Der Kopf 17 der Lagerhülse 15 ragt in die Lagerkammer 25 hinein und steht mit seinen einander gegenüberliegenden Seitenflächen den Lagerscheiben 28, 29 gegenüber. Die Seitenflächen bilden Anschlagflächen aus, die bei einer Verdrehung des Femurteils 3 um die durch die Lagerhülse 15 definierte Drehachse relativ zum Tibiateil 2 an einer der Lagerscheiben 28 beziehungsweise 29 anschlagen und damit die Drehbewegung begrenzen.

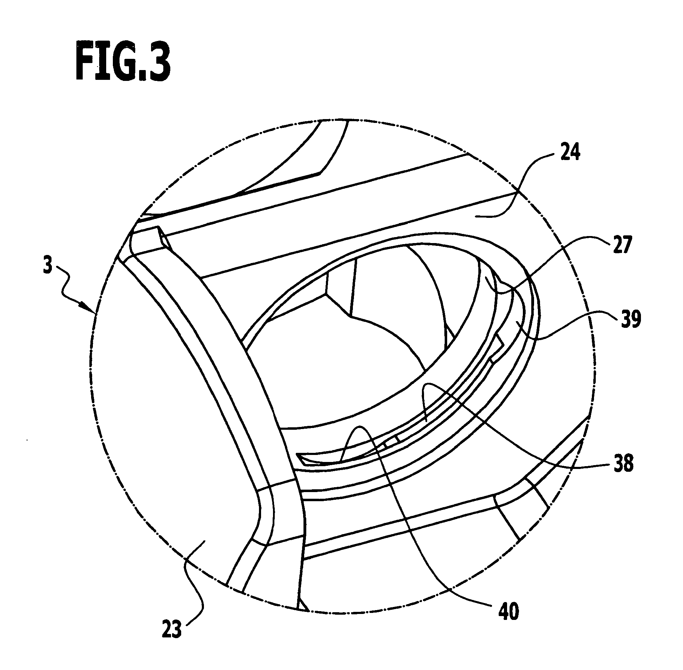

Die Lageröffnungen 26 sind beidseitig durch jeweils einen Deckel 36 verschließbar. Dazu weisen die Lageröffnungen 26 jeweils zwei diametral einander gegenüberliegende, sich über einen Umfangswinkel von etwa 90° erstreckende Nuten 38 in der Innenwand der Lageröffnungen 26 auf, und zwar unmittelbar neben dem äußeren Ende der Lageröffnungen 26 (Figur 3). Diese Nuten 38 sind in radialer Richtung nach innen offen und weisen an einem Ende eine zur Außenseite hin in axialer Richtung offene Einstecköffnung 39 auf, in diesem Bereich ist einfach die äußere Seitenwand der Nut 38 über die Querschnittsfläche der Einstecköffnung 39 offen.

Außerdem findet sich an dem der Einstecköffnung 39 gegenüberliegenden Ende der Nut 38 im Boden der Nut eine Vertiefung 40. Sowohl die Einstecköffnung als auch die Vertiefung sind auf ihrer Unterseite kreisbogenförmig begrenzt, und die Vertiefung 40 geht im Übergangsbereich zum Boden der Nut 38 unter einem Winkel in diesen über, der deutlich unter 45° liegt, im Übergangsbereich ist also eine relativ flache Stufe vorhanden.

Jeder Deckel 36 umfaßt eine kreisförmige Außenplatte 41, die an ihrer Innenseite einen zentralen, kreisförmigen Zapfen 42 trägt.

Auf diesen Zapfen 42 ist eine Federplatte 43 drehfest aufgesetzt, so daß der Zapfen 42 durch eine zentrale, kreisförmige Öffnung 44 in der Federplatte 43 hindurchtritt. Die Federplatte 43 umgibt den Zapfen 42 mittels eines ringförmigen Trägers 45, an den auf zwei gegenüberliegenden Seiten im Abstand zu diesem sich über einen Umfangswinkel von etwa 90° erstreckende Federarme 46, 47 angeformt sind. Diese tragen jeweils an ihrem freien Ende einen radial nach außen vorspringenden Vorsprung 48, 49, der außenseitig eine bogenförmige Kontur aufweist, die der bogenförmigen Kontur der Einstecköffnungen 39 und der Vertiefung 40 entspricht.

Auf der Außenseite des Deckels 36 sind zwei einander gegenüberliegende Vertiefungen 50, 51 eingearbeitet, diese dienen als Ansatzfläche für ein Drehwerkzeug oder zum manuellen Ergreifen und sind so angeordnet, daß manuell oder mit Hilfe eines Drehwerkzeuges der Deckel um seine Mittelachse verdreht werden kann.

Die Deckel 36 kann in die beidseitigen Lageröffnungen 26 von außen her unverlierbar eingesetzt werden. Dazu wird der Deckel zunächst axial so in die Lageröffnung 26 eingeschoben, daß die beiden Vorsprünge 48 und 49 durch die Einstecköffnung 39 in die Nut 38 eingeschoben werden können, anschließend wird der Deckel um etwa 90° gedreht. Dabei gleiten die Vorsprünge 48, 49 in den Nuten 38 entlang und werden in diesen geführt, bis sie in die Vertiefungen 40 am Ende der Nuten gelangen. In diese schnappen die Vorsprünge 48, 49 elastisch ein, da sie von den Federarmen 46, 47 elastisch radial nach außen gedrückt werden. In dieser Lage befindet sich der Deckel in Schließstellung, ist in axialer Richtung in der Nut festgelegt und auch in Umfangsrichtung durch das Einschnappen der Vorsprünge 48, 49 in die Vertiefung 40. Durch Drehen des Deckels in entgegengesetzter Richtung kann jedoch unter Überwindung eines bestimmten Drehmomentes jeder Vorsprung 48, 49 wieder aus der Vertiefung 40 ausgehoben werden, dabei werden die Federarme 46, 47 elastisch radial nach innen verbogen.

Die Deckel verschließen in der Schließstellung die Lageröffnungen 26 dicht und können auch dazu dienen, die Lagerwelle 27 in axialer Richtung festzulegen, diese liegt dann an den zentralen Zapfen 42 des Deckels 36 an.

## Patentansprüche

1. Knieendoprothese (1) mit einem Tibiateil (2) und einem Femurteil (3), die über ein Scharniergelenk miteinander schwenkbar verbunden sind, welches eine in zwei seitlichen Lageröffnungen des Femurteils (3) gelagerte Lagerwelle (27) und diese Lageröffnungen (26) außenseitig verschließende Deckel umfaßt, **dadurch gekennzeichnet, daß** die Deckel (36) mittels eines Bajonettverschlusses lösbar in der jeweiligen Lageröffnung (26) gehalten sind und zwischen einer Freigabestellung, in der sie aus der Lageröffnung (26) in axialer Richtung herausziehbar beziehungsweise in diese einschiebbar sind, und einer Schließstellung, in der sie in der Lageröffnung (26) in axialer Richtung festgelegt sind, verdrehbar sind.

2. Knieendoprothese nach Anspruch 1, **dadurch gekennzeichnet, daß** der Bajonettverschluß mindestens zwei sich über einen Teil des Umfangs längs des Randes der Lageröffnung (26) erstreckende, in radialer Richtung zur Innenseite der Lageröffnung (26) hin offene Nuten (38) umfaßt, die in axialer Richtung an einem Ende außenseitig eine Einführöffnung (39) aufweisen, und daß der Deckel (36) für jede Nut (38) einen radialen Vorsprung (48, 49) trägt, der beim winkelrichtigen Aufsetzen des Deckels (36) auf die Lageröffnung (26) in dessen FreigabeStellung in axialer Richtung durch die Einführöffnung (39) einer Nut (38) in die Ebene der Nut (38) vorschiebbar ist und beim Verdrehen des Deckels (36) in die Nut (38) eintritt und in dieser verschoben wird.

3. Knieendoprothese nach Anspruch 2, **dadurch gekennzeichnet, daß** zwei derartige Nuten (38) an der Lageröffnung (26) diametral gegenüberliegen und der Deckel (36) zwei entsprechende radiale Vorsprünge (48, 49) trägt.

4. Knieendoprothese nach Anspruch 2 oder 3, **dadurch gekennzeichnet, daß** sich die Nuten (38) über einen Umfangswinkel von maximal 90° erstrecken.

5. Knieendoprothese nach einem der voranstehenden Ansprüche, **dadurch gekennzeichnet, daß** der Deckel (36) und die Lageröffnung (26) in der Schließstellung durch ein elastisch in eine Rastausnehmung (40) eintauchendes Rastglied (48, 49) gegen eine Verdrehung lösbar gesichert sind.

6. Knieendoprothese nach Anspruch 5, **dadurch gekennzeichnet, daß** das Rastglied durch die radialen Vorsprünge (48, 49) und die Rastausnehmungen durch Vertiefungen (40) im Boden der Nuten (38) an deren den Einführöffnungen (39) abgewandten Enden gebildet werden.

7. Knieendoprothese nach Anspruch 6, **dadurch gekennzeichnet, daß** die Rastglieder (48, 49) und die Rastausnehmungen (40) einen komplementären, bogenförmigen Querschnitt aufweisen.

8. Knieendoprothese nach einem der Ansprüche 5 bis 7, **dadurch gekennzeichnet, daß** die Rastglieder (48, 49) an elastisch verformbaren Haltearmen (46, 47) angeordnet und in radialer Richtung federnd nach innen eindrückbar sind.

9. Knieendoprothese nach Anspruch 8, **dadurch gekennzeichnet, daß** die Haltearme (46, 47) an einem Träger (45) außenseitig angeformt sind, der an der Innenseite des Deckels (36) festgelegt ist.

10. Knieendoprothese nach Anspruch 9, **dadurch gekennzeichnet, daß** der Träger (45) als Ring ausgebildet ist.

11. Knieendoprothese nach Anspruch 10, **dadurch gekennzeichnet, daß** der Ring drehfest auf einen Haltezapfen (42) auf der Innenseite des Dekkels (36) aufgesteckt ist.

12. Knieendoprothese nach einem der Ansprüche 9 bis 11, **dadurch gekennzeichnet, daß** sich die Haltearme (46, 47) im wesentlichen konzentrisch zum Rand des Deckels (36) über einen Teil seines Umfanges erstrecken.

13. Knieendoprothese nach einem der Ansprüche 9 bis 12, **dadurch gekennzeichnet, daß** der Träger (45), die Haltearme (46, 47) und die Rastvorsprünge (48, 49) Teile eines einstückig ausgebildeten Bauteils (43) sind.

14. Knieendoprothese nach Anspruch 13, **dadurch gekennzeichnet, daß** der Träger (45), die Haltearme (46, 47) und die Rastvorsprünge (48, 49) Teile eines plattenförmigen Materialstückes (43) sind.

15. Knieendoprothese nach einem der voranstehenden Ansprüche, **dadurch gekennzeichnet, daß** der Deckel (36) auf seiner Außenseite außermittige Vertiefungen (50, 51) zum manuellen Erfassen oder zum Ansetzen eines Drehwerkzeuges aufweist.

16. Knieendoprothese nach einem der voranstehenden Ansprüche, **dadurch gekennzeichnet, daß** der Deckel (36) mit seiner Außenseite die Lageröffnung (27) bündig verschließt.

## Claims

1. A knee endoprosthesis (1) with a tibial part (2) and a femoral part (3) which are pivotably connected to one another by a hinge joint comprising a bearing shaft (27) mounted in two lateral bearing openings in the femoral part (3), and covers which externally close these bearing openings, **characterised in that** the covers (36) are detachably held in the respective bearing opening (26) by means of a bayonet fixing and are rotatable between a release position, in which they can be withdrawn from or pushed into the bearing opening (26) in the axial direction, and a closing position in which they are secured in the bearing opening (26) in the axial direction.

2. A knee endoprosthesis according to claim 1, **characterised in that** the bayonet fixing comprises at least two grooves (38) which extend over part of the circumference along the edge of the bearing opening (26) and are open towards the inside of the bearing opening (26) in a radial direction and which have an insertion opening (39) on the outside at one end in the axial direction, and **in that** the cover (36) for each groove (38) carries a radial projection (48, 49) which, when the cover (36) is placed at the correct angle onto the bearing opening (26) in its release position, can be pushed forwards in the axial direction through the insertion opening (39) of a groove (38) into the plane of the groove (38) and, during rotation of the cover (36), enters the groove (38) and is displaced therein.

3. A knee endoprosthesis according to claim 2, **characterised in that** two such grooves (38) lie diametrically opposite one another in the bearing opening (26), and the cover (36) carries two corresponding radial projections (48, 49).

4. A knee endoprosthesis according to claim 2 or 3, **characterised in that** the grooves (38) extend over a circumferential angle of a maximum of 90°.

5. A knee endoprosthesis according to any one of the preceding claims, **characterised in that** the cover (36) and the bearing opening (26) are releasably secured against rotation in the closing position by a locking member (48, 49) extending resiliently into a locking recess (40).

6. A knee endoprosthesis according to claim 5, **characterised in that** the locking member is formed by the radial projections (48, 49), and the locking recesses are formed by depressions (40) in the base of the grooves (38) at their ends remote from the insertion openings (39).

7. A knee endoprosthesis according to claim 6, **characterised in that** the locking members (48, 49) and the locking recesses (40) have a complementary, curved cross-section.

8. A knee endoprosthesis according to any one of claims 5 to 7, **characterised in that** the locking members (48, 49) are arranged on resiliently deformable holding arms (46, 47) and can be pushed resiliently inwards in a radial direction.

9. A knee endoprosthesis according to claim 8, **characterised in that** the holding arms (46, 47) are externally formed on a carrier (45) which is fastened to the inside of the cover (36).

10. A knee endoprosthesis according to claim 9, **characterised in that** the carrier (45) is formed as a ring.

11. A knee endoprosthesis according to claim 10, **characterised in that** the ring is mounted in a rotationally fixed manner on a holding pin (42) on the inside of the cover (36).

12. A knee endoprosthesis according to any one of claims 9 to 11, **characterised in that** the holding arms (46, 47) extend substantially concentrically with the edge of the cover (36) over part of its circumference.

13. A knee endoprosthesis according to any one of claims 9 to 12, **characterised in that** the carrier (45), the holding arms (46, 47) and the locking projections (48, 49) are parts of a one-piece component (43).

14. A knee endoprosthesis according to claim 13, **characterised in that** the carrier (45), the holding arms (46, 47) and the locking projections (48, 49) are parts of a plate-shaped material piece (43).

15. A knee endoprosthesis according to any one of the preceding claims, **characterised in that** the outside of the cover (36) is provided with eccentric depressions (50, 51) to enable it to be gripped manually or for application of a turning tool.

16. A knee endoprosthesis according to any one of the preceding claims, **characterised in that** the outside of the cover (36) closes the bearing opening (27) so as to be flush therewith.

## Revendications

1. Endoprothèse du genou (1) comportant un élément tibial (2) et un élément fémoral (3) qui sont assemblés de manière pivotante l'un à l'autre par l'intermédiaire d'une articulation à charnière, laquelle comporte un axe de palier (27), logé dans deux orifices d'appui latéraux de l'élément fémoral (3), et des couvercles obturant de l'extérieur ces orifices d'appui (26), **caractérisée en ce que** les couvercles (36) sont maintenus de manière amovible par un assemblage à baïonnette dans l'orifice d'appui (26) respectif et sont aptes à tourner entre une position de dégagement, dans laquelle ils peuvent être retirés dans le sens axial hors de l'orifice d'appui (26) ou être insérés dans celui-ci, et une position de fermeture, dans laquelle ils sont fixés dans le sens axial dans l'orifice d'appui (26).

2. Endoprothèse du genou selon la revendication 1, **caractérisée en ce que** l'assemblage à baïonnette comporte au moins deux rainures (38) ouvertes dans le sens radial vers la face intérieure de l'orifice d'appui (26), lesquelles s'étendent sur une partie du pourtour le long du bord de l'orifice d'appui (26) et comportent au niveau d'une extrémité dans le sens axial une ouverture d'introduction (39) sur le côté extérieur, et **en ce que** le couvercle (36) porte pour chaque rainure (38) une saillie radiale (48, 49) qui, lorsque le couvercle (36) est en position angulaire correcte sur l'orifice d'appui (26) dans la position de dégagement de celui-ci, peut être avancée dans le sens axial à travers l'ouverture d'introduction (39) d'une rainure (38) dans le plan de la rainure (38) et, moyennant une rotation du couvercle (36), s'engage dans la rainure (38) et est déplacée dans celle-ci.

3. Endoprothèse du genou selon la revendication 2, **caractérisée en ce que** deux rainures (38) de ce type sont diamétralement opposées sur l'orifice d'appui (26) et le couvercle (36) porte deux saillies radiales (48, 49) correspondantes.

4. Endoprothèse du genou selon la revendication 2 ou 3, **caractérisée en ce que** les rainures (38) s'étendent sur un angle inscrit de 90° maximum.

5. Endoprothèse du genou selon l'une quelconque des revendications précédentes, **caractérisée en ce que**, dans la position de fermeture, le couvercle (36) et l'orifice d'appui (26) sont immobilisés en rotation de manière amovible par un élément de blocage (48, 49) s'engageant de manière élastique dans un évidement de blocage (40).

6. Endoprothèse du genou selon la revendication 5, **caractérisée en ce que** l'élément de blocage est formé par les saillies radiales (48, 49) et les évidements de blocage sont formés par des creux (40) dans le fond des rainures (38) au niveau des extrémités de celles-ci opposées aux ouvertures d'introduction (39).

7. Endoprothèse du genou selon la revendication 6, **caractérisée en ce que** les éléments de blocage (48, 49) et les évidements de blocage (40) ont une section courbe complémentaire.

8. Endoprothèse du genou selon l'une quelconque des revendications 5 à 7, **caractérisée en ce que** les éléments de blocage (48, 49) sont agencés sur des bras de fixation (46, 47) élastiquement déformables et peuvent être poussés élastiquement vers l'intérieur dans le sens radial.

9. Endoprothèse du genou selon la revendication 8, **caractérisée en ce que** les bras de fixation (46, 47) sont formés du côté extérieur sur un support (45), qui est fixé contre la face intérieure du couvercle (36).

10. Endoprothèse du genou selon la revendication 9, **caractérisée en ce que** le support (45) est réalisé sous forme de bague.

11. Endoprothèse du genou selon la revendication 10, **caractérisée en ce que** la bague est emmanchée immobile en rotation sur un téton de fixation (42) sur la face intérieure du couvercle (36).

12. Endoprothèse du genou selon l'une quelconque des revendications 9 à 11, **caractérisée en ce que** les bras de fixation (46, 47) s'étendent sensiblement concentriquement par rapport au bord du couvercle (36) sur une partie du pourtour de celui-ci.

13. Endoprothèse du genou selon l'une quelconque des revendications 9 à 12, **caractérisée en ce que** le support (45), les bras de fixation (46, 47) et les saillies de blocage (48, 49) sont des éléments d'une pièce (43) réalisée d'un seul tenant.

14. Endoprothèse du genou selon la revendication 13, **caractérisée en ce que** le support (45), les bras de fixation (46, 47) et les saillies de blocage (48, 49) sont des éléments d'une partie de matériau (43) en forme de plaque.

15. Endoprothèse du genou selon l'une quelconque des revendications précédentes, **caractérisée en ce que** le couvercle (36) sur sa face extérieure comporte des creux (50, 51) excentrés, pour le saisir manuellement ou pour y appliquer un outil tournant.

16. Endoprothèse du genou selon l'une quelconque des revendications précédentes, **caractérisée en ce que** le couvercle (36) obture à fleur l'orifice d' appui (26) avec sa face extérieure.
